Europäisches Patentamt

(19) European Patent Office

Office européen des brevets

(11) EP 0 656 775 B1

## (12) EUROPEAN PATENT SPECIFICATION

(45) Date of publication and mention
of the grant of the patent:
**29.12.1997 Bulletin 1997/52**

(21) Application number: **93918020.4**

(22) Date of filing: **13.08.1993**

(51) Int. Cl.$^6$: **A61K 31/135**, A61K 9/20,
A61K 47/18

(86) International application number:
**PCT/GB93/01722**

(87) International publication number:
**WO 94/04138 (03.03.1994 Gazette 1994/06)**

(54) **SUSTAINED RELEASE TABLETS CONTAINING BUPROPION**

TABLETTEN MIT VERZÖGERTER WIRKSTOFFABGABE ENTHALTEND BUPROPION

COMPRIMES A LIBERATION PROLONGEE CONTENANT DU BUPROPIONE

(84) Designated Contracting States:
**AT BE CH DE DK ES FR GB GR IE IT LI LU MC NL
PT SE**

(30) Priority: **14.08.1992 GB 9217295**

(43) Date of publication of application:
**14.06.1995 Bulletin 1995/24**

(73) Proprietor:
**THE WELLCOME FOUNDATION LIMITED
Greenford, Middlesex UB6 0NN (GB)**

(72) Inventors:
• **LUDWIG, Jennie Sue Galloway
Greenville, NC 27858 (US)**
• **SUTTON, Joel Elmore, Jr.
Greenville, NC 27858 (US)**

• **BASS, William Leroy, Jr.
Farmville, NC 27828 (US)**

(74) Representative:
**Stott, Michael John et al
Glaxo Wellcome plc,
Glaxo Wellcome House,
Berkeley Avenue
Greenford, Middlesex UB6 0NN (GB)**

(56) References cited:
**EP-A- 0 171 457**          **EP-A- 0 467 488**
**WO-A-92/19226**           **US-A- 3 819 706**
**US-A- 3 885 046**         **US-E- R E33 994**

• **Dow Manual "Formulating for controlled release
with Methocel Premium cellulose ether", Dow
Copyright 1987, p.17, p.19**

**Description**

Bupropion hydrochloride (Wellbutrin[R]) is a marketed antidepressant. It is chemically known as (±)-2-(tert-butylamino)-3'-chloropropiophenone hydrochloride. (See U.S. Patents 3,819,706 and 3,885,046). Also see Merck Index, Eleventh Edition, entry No. 1488.

In usage, bupropion hydrochloride is sold in the form of an instant release tablet wherein greater than 75% of bupropion hydrochloride is released from the tablet dissolution media in 45 minutes (See 1993 Physicians Desk Reference (PDR), pages 842 to 844). The PDR indicates bupropion hydrochloride as presently sold in an instant release tablet as being associated with seizures in approximately 0.4% (4/1000) patients treated at doses of up to 450 mg per day. In studies to date, the risk of seizures seem to be strongly associated, in part, with use of instant release tablets.

In order to reduce the seizure rate, it has been determined after experimentation that a controlled sustained release of bupropion hydrochloride should be employed.

Prior art with respect to this invention is to be found in U.S. Patent No. 4,687,660 which discloses controlled sustained release tablets containing bupropion hydrochloride.

The present invention provides an improved product in that it provides sustained release of the active ingredient as well as ease of manufacture over that disclosed in U.S. Patent 4,687,660.

After almost three years of work and testing, the present invention is now able to provide a controlled sustained release, sometimes also referred to as a sustained release (SR) tablet with improved properties and which has a shelf life over one year (i.e., there is less than 10% loss most preferably less than about 5% loss breakdown), of buproprion hydrochloride) in one year of storage at room temperature 15 to 25°C (59 to 77°F) at 35 to 60% relative humidity. For example, with a tablet containing 100 mg of bupropion hydrochloride (label strength) the tablet will preferably contain no less than about 95mg of bupropion hydrochloride after one year in storage (shelf life). As used herein, the term "tablet" includes the term "caplet", which is the word used to describe a tablet termed substantially in the shape of a capsule.

The oral administration of the controlled release tablets of this invention to treat depression in humans will be under the jurisdiction of the physician; however, tablets for a 75kg (150 pound) human will ordinarily be given one to three times per day to provide a total daily dosage of 100 to 450mg per day for a time period that the human patient requires same as determined by a physician. The tablets are swallowed by the human in the normal manner with the aid of water or other liquid. The tablets herein preferably have a round biconvex shape (which is preferred); however, this may be varied depending on the creativity of the tablet designer. With the tablets of this invention it is now possible to dose one or two times per day rather than the three times a day as presently done with the currently marketed product.

The present invention provides a sustained release tablet comprising bupropion hydrochloride and hydroxypropyl methylcellulose, the amount of hydroxypropyl methylcellulose to the amount of bupropion hydrochloride being from 0.19:1 to 1.1:1 respectively and the tablet being capable in water of releasing from 20 to 60 percent of the total amount of bupropion hydrochloride in 1 hour, from 50 to 95 percent of the total amount of bupropion hydrochloride in 4 hours and not less than 75 percent of the total amount of bupropion hydrochloride in 8 hours. Cysteine hydrochloride or glycine hydrochloride may also be included. Cysteine hydrochloride is more preferable than glycine hydrochloride, since it unexpectedly causes less discoloration to the tablet core. The amount of bupropion hydrochloride in each tablet is typically from 25mg to 500mg, with the tablet dose being more typically 50mg, 100mg or 150mg.

Methocel[R] is the brand name for hydroxypropyl methylcellulose (HPMC) from Dow Chemical. Other companies also supply HPMC.

Specific embodiments of the invention will now be described by way of example with reference to the accompanying drawings in which:-

Fig. 1 is a top view of the preferred tablet shape of this invention:

Fig. 2 is a side view of the tablet of Fig. 1;

Fig. 3 is a sectional view taken along line 3-3 in Fig. 1;

Fig. 4 is a graph showing plasma bupropion (as the free base) concentrations in nanograms/ml in adult male humans following a single dose of two 50mg bupropion HCl (SR) tablets, divided by two over 24 hours from time of administration provided by the 50mg tablet example of this invention;

Fig. 5 is a graph showing plasma bupropion (as the free base) concentrations in nanograms/ml in adult male humans following a single dose of one 100mg bupropion HCl (SR) tablets over 24 hours provided by the 100mg tablet example of this invention; and

Fig. 6 is a graph showing plasma bupropion (as the free base) concentrations in nanograms/ml in adult male humans following a single 150mg tablet over 24 hours provided by the 150mg (SR) tablet example of this invention.

In order to prepare the controlled sustained release (SR) tablets of this invention, particles of bupropion hydrochloride are preferably blended with microcrystalline cellulose and hydroxypropyl methylcellulose (Methocel) to form an admixture of blended powders. A 20 mesh sieve is conveniently used to screen the bupropion hydrochloride particles, cellulose particles and hydroxypropyl methylcellulose particles prior to blending the ingredients. Then the cysteine hydrochloride or glycine hydrochloride is dissolved in water to form a granulating solution. Thereafter, the granulating solution is preferably sprayed onto the blended powders, which are then dried. A lubricant such as magnesium stearate is added and intermixed (blended) with the blended powders having the granulating solution dried thereon. Other suitable fillers include the following: lactose, starch. Microcrystalline cellulose is also preferably added to the product to provide compressibility. Compression of the mixture in a punch and die is then used to form the tablet core.

Thereafter, the tablet (sometimes referred to as the core) is preferably film coated with a colored coating such as a Opadry Purple, Opadry Blue or Opadry White for identification, taste masking, and appearance purposes to provide a film coated tablet.

The film coating does not substantially affect the release rate of the bupropion hydrochloride from the tablet, since the coating is instant release which rapidly dissolves in the stomach. The outer film coating is typically 0.03mm to 0.10 mm in thickness.

In the practice of this invention, for every part by weight of bupropion hydrochloride, the amount of hydroxypropyl methylcellulose is 0.19 to 1.1 and more preferably 0.26 to 0.68 parts by weight and the amount of cysteine hydrochloride or glycine hydrochloride is 0.02 to 0.27 and more preferably 0.05 to 0.16 parts by weight. For example, in a tablet containing 100mg of bupropion hydrochloride, the amount of hydroxypropyl methylcellulose is 19mg to 110mg and most preferably, 26.7 to 68mg and the amount of cysteine hydrochloride or glycine hydrochloride is 2.7mg to 27mg and most preferably 5mg to 16.2mg.

The surface area to volume of the non film-coated or core portion of the tablets herein is important in maintaining the appropriate controlled sustained release rates.

The ratio of the non film coated tablet (sometimes referred to as the core) surface area to tablet volume is preferably 3:1 to 25:1cm$^{-1}$ and more preferably 7:1 to 16:1cm$^{-1}$ for tablets of 50, 100 and 150mg bupropion hydrochloride content. For tablets of 50mg bupropion HCl content the ratio of the tablet surface area to tablet volume is most preferably 13:1 to 16:1cm$^{-1}$ and for the 100mg bupropion HCl content tablet the ratio is most preferably 9:1 to 12:1cm$^{-1}$ and for the 150mg bupropion HCl content tablet the ratio is most preferably 7:1 to 10:1cm$^{-1}$.

The release rate of bupropion hydrochloride from the sustained release (SR) tablets disclosed (whether or not film coated) herein in distilled water is preferably as follows;

about 20% to about 60% (most preferably 25 to 50%) in 1 hour;

about 50% to 95% (most preferably 60 to 95%) in 4 hours; and

not less than about 75% (most preferably not less than 80%) in 8 hours. For the bupropion HCl 50mg content tablet the release rate of bupropion HCl is preferably 30 to 50% in 1 hour, 70 to 95% in 4 hours and not less than 80% in 8 hours. For the 100mg bupropion HCl content tablet the release rate is preferably 25 to 45% in 1 hour, 60 to 85% in 4 hours and not less than 80% in 8 hours. With the 150mg content tablet the release rate is preferably 25 to 45% in 1 hour, 60 to 75% in 4 hours and not less than 85% in 8 hours.

The test conditions and apparatus to determine the drug release (dissolution) rates of buproprion hydrochloride are as follows:

Apparatus: USP Rotating Paddle (Apparatus II);
Stirring Rate: 50 rpm;
Sample Size: a single unweighted tablet per vessel;
Temperature: 37°C ± 0.5°C
Medium for Dissolution of tablet: 900 ml of distilled water.

The test for dissolution (release rates) is performed as specified below in the U.S. Pharmacopoeia under "Drug Release" and the medium is sampled at 1.4 and 8 hours.

Sample Preparation: Withdraw a measured portion of the dissolution medium not to exceed 10ml at each sampling point without replacement. Filter the sample through a LID/X GMF 0.45 micron filter or a Zymark 10 micron filter (or equivalent).

Standard Preparation: Accurately weigh approximately 50mg of Bupropion Hydrochloride Reference Standard and transfer into 900ml volumetric flask. Dissolve in and dilute to volume with water and mix (nominal concentration: 0.0556mg bupropion hydrochloride per ml).

Instrumental Conditions:

Instrument:       An appropriate HPLC equipped with a 10-$\mu$l sampling loop
Column:           BDS Hypersil C18.5 micron (50x4.6mm) column, or SUPELCOSIL LC-18-DB, 3 micron (33x4.6mm), or equivalent
Flow Rate:        2.0ml/min
Detection:        UV. 224nm. 0.1 AUFS or an appropriate AUFS setting
Mobile Phase:     Methanol:pH 7.0 phosphate buffer (65:35) appropriately filtered and degassed.

System Suitability Test: Replicate injections of the Standard Preparation give relative standard deviations for peak retention times and peak responses no greater than 2 percent. The tailing factor (T.USP) for the bupropion peak is not greater than 2.5.

Ph 7.0 Phosphate Buffer Preparation: Transfer 27.22g of monobasic potassium phosphate into a 1000-ml volumetric flask. Dissolve in and dilute to volume with water and mix. Transfer 250ml of this solution into a 1000-ml of 0.291 M sodium hydroxide, dilute to volume with water and mix.

Calculation

Step 1 - Determination of Bupropion Hydrochloride Concentration

$$\% \text{ Nominal Bupropion Hydrochloride (0.0556mg/ml)} = \frac{\text{SPL Pk Response}}{\text{STD Pk Response}} \times \frac{\text{STD Wt}}{900} \times \frac{900}{\text{L.S}} \times 100$$

Step II - Sampling Volume Corrections Perform the following calculations to adjust for sample removal. (Evaporation is insignificant over an 8-hour period when using the Zymate II Automated Dissolution Testing System).

$$\% \text{ L.S. Bupropion Hydrochloride released (50mg tablet)} =$$

$$\frac{900 - nv}{900} \times \% \text{ nominal bupropion hydrochloride (sample (n-1))} =$$

$$\frac{v \sum_{i=1}^{n}}{900} \% \text{ nominal bupropion hydrochloride (sample (i))}$$

where

n       = number of previous samples taken
v       = volume of sample taken each time

These calulations can be performed using the BASIC program "DISTAB".

The tablet also preferably includes as other ingredients. e.g., lubricating agents for ease in manufacture and fillers for ease of manufacturing and to bulk the tablet to provide the desired size.

The tablet is also preferably coated with an instant release coating for appearance, taste masking, and product identification e.g. Opadry Purple, Blue or White, which will dissolve in the stomach and not substantially affect bupropion hydrochloride release rates and bupropion hydrochloride stability.

Hydroxypropyl Methylcellulose 2910. USP used in the examples, conforms to 28.0 to 30.00% methoxyl substitution and 7.0 to 12.0% hydroxypropoxyl substitution. The preferred nominal viscosity of 2% solution in water is not less than 3,000 centipoise and not more than 5,600 centipoise. It is supplied by Dow Chemical Company, Midland, MI as Methocel E4M premium CR.

Microcrystalline cellulose, NF supplied by FMC Corporation as Avicel PH 102, has an average particle size of 90 micrometers with particle size specification of 8% $\geq$ 60 mesh and 45% $\leq$ 200 mesh.

Opadry[R] Purple YS-1-4845 is supplied by Colorcon. inc., contains FD&C Red No. 40 aluminum lake, hydroxypropyl methycellulose, titanium dioxide, polyethylene glycol, olysorbate 80, and FD&C Blue No. 2 Aluminum Lake.

Opadry[R] Blue YS-1-4282 is supplied Colorcon. Inc.. contains FD&C Blue No. 1 aluminum lake, hydroxypropyl methycellulose, titanium dioxide, polyethylene glycol, and polysorbate 80.

Opadry[R] White YS-1-7059 is supplied by Colorcon. Inc., contains hydroxypropyl methylcellulose, titanium dioxide, and polyethylene glycol.

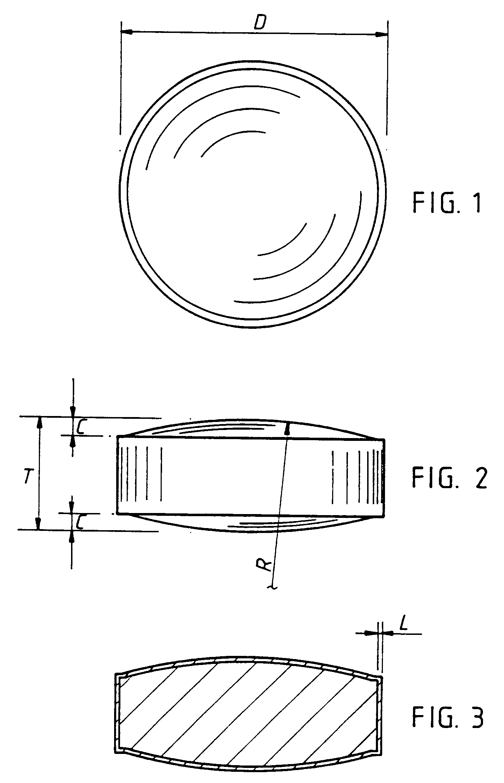

Reference should now be had to Figs. 1 to 3 for a description of the preferred form of the biconvex film coated tablets of this invention which contain as the active ingredient (drug) 50mg, 100mg or 150mg of bupropion HCl. The tablet (core) is shown at 10 and the film coating is shown at 11 in Fig.3

Tablets having the following dimensions as set forth below for the 50mg, 100mg and 150mg bupropion HCl content tablets in which the letters D (diameter), T (thickness), L (land), C (cap depth) and the R (radius of curvature of the biconvex region) are shown in Figs. 1 to 3.

|  | 50mg | 100mg | 150mg |
|---|---|---|---|
| R in mm (inches) | 10.41 (.410) | 12.15 (.4783) | 15.34 (.604) |
| D in mm (inches) | 7.40 (.2913) | 9.40 (.3701) | 11.00 (.4331) |
| C in mm (inches) | 0.66 (.026) | 0.91 (.036) | 0.99 (.039) |
| T in mm | 3.40 | 4.32 | 4.65 |
| L in mm (inches) | 0.51 (.002) | 0.076 (.003) | 0.076 (.003) |

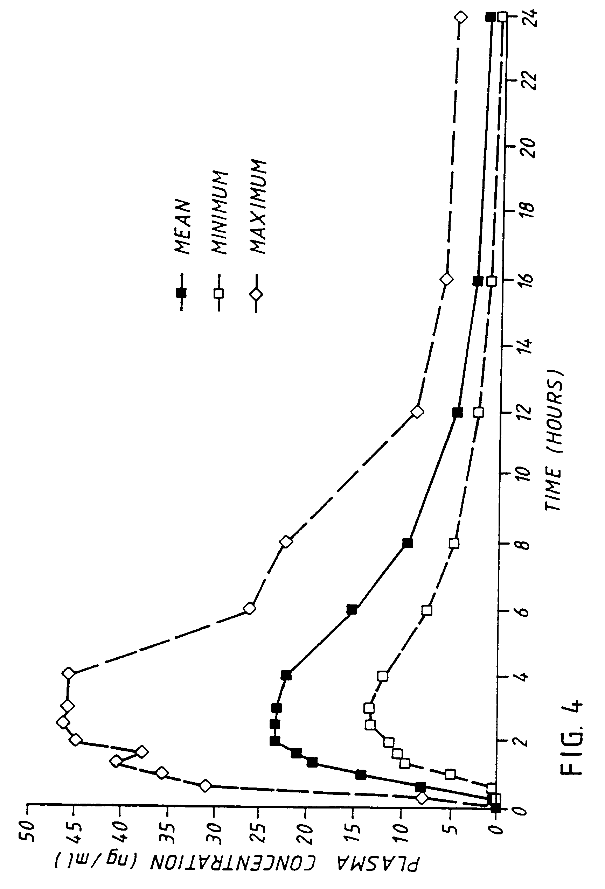

Reference now should be had to Figs. 4, 5 and 6 which illustrate plasma bupropion levels, as the free base i.e. the compound bupropion itself (see the Merck Index, Eleventh Edition entry No. 1488) after oral administration to adult males (humans) of 50mg, 100mg and 150mg bupropion HCl film coated tablets of the examples herein. It is to be understood that because of the nature of the film coating, the release rate will be substantially the same whether or not the tablets are film-coated and, therefore, the data is also representative of non film coated tablets disclosed herein.

In particular, Fig. 4 represents plasma levels (divided by 2) of bupropion after oral dosage with two 50mg SR tablets in 21 male (human) patients 18 to 40 years of age weighing 66 to 92kg (132 to 184 lbs). The maximum level shown is detected in the blood plasma of at least one of the patients and the minimum being that detected in the blood plasma of at least one of the patients and the mean being the average of all measurements of all participants.

The bupropion plasma levels for the 50mg tablet dosing measured over 24 hours as in this figure were done as described in the radioimmunoassay test set forth in the article Radioimmunoassay And Pharmacokinetic Profile of Bupropion In The Dog by Robert F. Butz *et al*. published in the Journal of Pharmacology and Experimental Therapeutics. Vol. 217. No. 3 (1981).

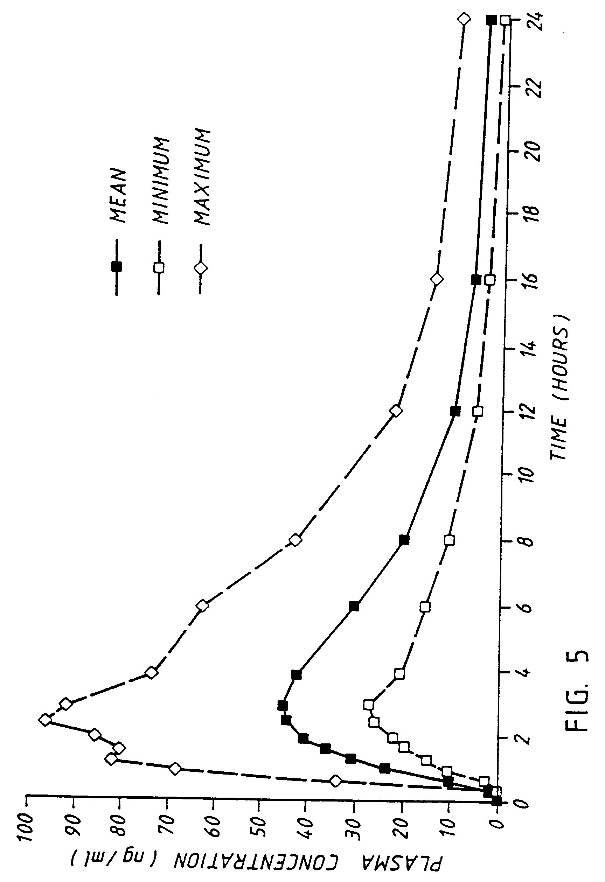

Fig. 5 is a repeat with respect to Fig. 4; however a single oral dose of a 100mg SR tablet of this disclosure's example was administered to the same group of males as those tested previously in Fig. 4; however, the bupropion plasma levels were not divided in two. The plasma levels were also measured at the time intervals shown using the test procedure described in the aforementioned Butz *et al*. article.

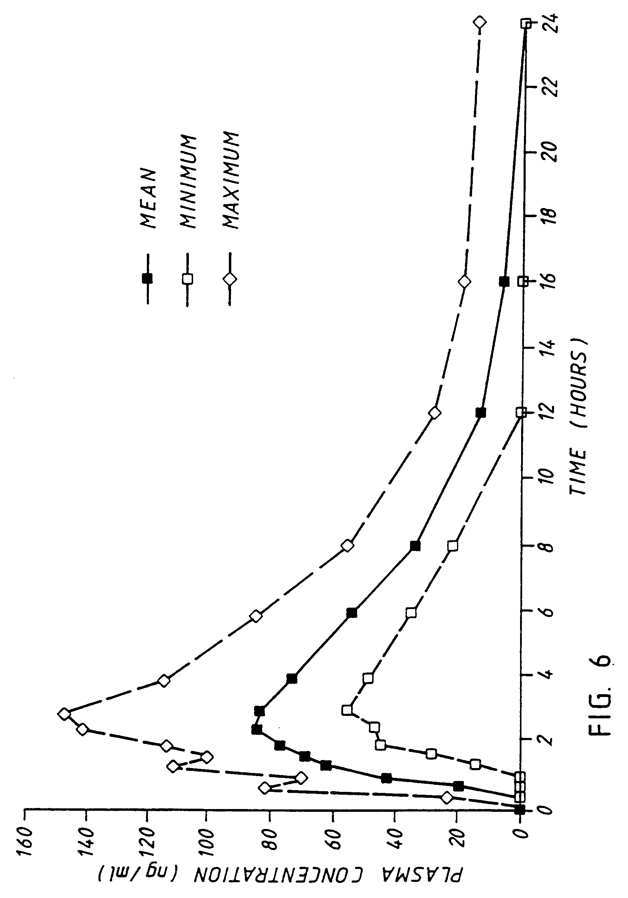

Fig. 6 represents oral dosing of 24 male (human) patients, ages 20 to 39 years, weight 68 to 98kg (136 to 196lbs). Each of the males was dosed once with a 150mg bupropion HCl SR tablet and bupropion plasma level measurements were made as shown on the plot of Fig. 6 at the time intervals shown to arrive at the maximum, minimum for any patient and the mean (average) for all patients in the test.

The measurements using the 150mg tablets of the examples were determined following the procedure described in the article entitled "Determination of Bupropion and Its Major Basic Metabolites in Plasma by Liquid Chromatography with Dual - Wavelength Ultraviolet Detection" by Thomas B. Cooper *et al*. published in Journal of Pharmaceutical Sciences. Vol. 73. No. 8. August 1984. The results were not divided by two as with the Fig. 4 results.

It should be understood that variations may occur in biological measurements from test to test. It should also be understood that the tablet measurements may change somewhat without departing from the spirit of the invention and that the tablet dimensions will also vary because of variability in materials, manufacturing processes, and tolerance achievable.

It should be understood that the outer coating on the core is preferred in this invention but is not a required feature of the invention herein.

## EXAMPLE 1

**PREPARATION OF 150MG BUPROPION HCL CONTENT TABLETS**

| CORE INGREDIENTS | mg/tablet | kg/batch |
|---|---|---|
| Bupropion Hydrochloride | 150.0 | 150.0 |
| Microcrystalline Cellulose, NF | 198.5 | 198.5 |
| Hydroxypropyl Methylcellulose 2910, USP | 40.00 | 40.00 |
| Cysteine Hydrochloride, USP | 7.50 | 7.50 |
| Magnesium Stearate, NF | 4.00 | 4.00 |
| Purified Water, USP | q.s. | q.s |
| | 400.0 mg | |
| Surface Area to Volume Ratio | $8.818cm^{-1}$ | |
| Volume | $0.3656cm^3$ | |
| Surface Area | $3.224cm^2$ | |
| COATING INGREDIENTS | | |
| Opadry Purple YS-1-4845 | 16.00 | 20.00kg* |
| Purified Water, USP | q.s | 180.00kg |
| Carnauba Wax, NF | 0.04 | 0.04kg |
| Coating thickness≈ 0.05 - 0.10mm for all tablets of the examples. "q.s." means sufficient quantity in the examples. | | |

*Includes 25% overage.

TYPICAL MANUFACTURING PROCEDURE AND ASSEMBLING PROCESS

Bupropion Hydrochloride Sustained-Release Tablets, 150mg Bupropion Hydrochloride per coated Tablet.

1. Sift and blend the following:

   bupropion hydrochloride
   microcrystalline cellulose
   hydroxypropyl methylcellulose

2. Dissolve the cysteine hydrochloride in the purified water.

3. Spray the solution from Step 2 onto the blended powders from Step 1 using a fluid bed granulator. Additional purified water may be used is necessary, to obtain proper granule wetness.

4. Dry the granules.

5. Add magnesium stearate to the granules and mill.

6. Blend the milled granules.

7. Compress the granules at approximately 400.0mg or a weight equivalent to 150mg bupropion hydrochloride on a rotary press fitted with 11.0mm round punches and dies, the upper and lower punches plain.

8. Prepare a film-coating suspension of Opadry Purple YS-1-4845 dispersed in purified water.

9. Spray the coating suspension onto the core tablets using suitable coating equipment until the desired weight gain is achieved.

10. Polish the coated tablets in the coating equipment using carnauba wax.

## EXAMPLE 2

## PREPARATION OF 100MG BUPROPION HC1 CONTENTS TABLETS

| CORE INGREDIENTS | mg/tablet | kg/batch |
|---|---|---|
| Bupropion Hydrochloride | 100.00 | 150.0 |
| Microcrystalline Cellulose, NF | 97.00 | 145.5 |
| Hydroxypropyl Methylcellulose 2910, USP | 54.00 | 81.00 |
| Cysteine Hydrochloride, USP | 16.20 | 24.30 |
| Magnesium Stearate, NF | 2.800 | 4.200 |
| Purified Water. USP | q.s | 100.0 |
| | $\overline{270.0mg}$ | |
| Surface Area to Volume Ratio | $10.89cm^{-1}$ | |
| Volume | $0.2766cm^3$ | |
| Surface Area | $3.013cm^2$ | |
| COATING INGREDIENTS | | |
| Opadry Blue YS-1-4282 | 10.00 | 20.00* |
| Purified Water, USP | q.s | 180.0 |
| Carnauba Wax, NF | 0.02667 | .040 |
| | $\overline{280.03mg}$ | |
| Coating thickness $\approx$ 0.05 - 0.10mm | | |

*Includes 33.3% overage

TYPICAL MANUFACTURING PROCEDURE AND ASSEMBLING PROCESS

Bupropion Hydrochloride Sustained-Release Tablets 100mg Bupropion Hydrochloride Per Coated Tablet.

1. Sift and blend the following:

    bupropion hydrochloride
    microcrystalline cellulose
    hydroxypropyl methylcellulose.

2. Dissolve the cysteine hydrochloride in the purified water.

3. Spray the solution from Step 2 onto the blended powders from Step 1 using a fluid bed granulator. Additional purified water may be used if necessary, to obtain proper granule wetness.

4. Dry the granules.

5. Add magnesium stearate to the granules and mill.

6. Blend the milled granules.

7. Compress the granules at approximately 270.0mg or a weight equivalent to 100mg bupropion hydrochloride on a rotary press fitted with 9.4mm round punches and dies, the upper and lower punches plain.

8. Prepare a film-coating suspension of Opadry Blue, YS-1-4282 dispersed in purified water.

9. Spray the coating suspension onto the core tablets using suitable coating equipment until the desired weight gain is achieved.

10. Polish the coated tablets in the coating equipment using carnauba wax.

## EXAMPLE 3

## PREPARATION OF 50MG BUPROPION HCL CONTENT TABLETS

| CORE INGREDIENTS | mg/tablet | kg/batch |
|---|---|---|
| Bupropion Hydrochloride | 50.0 | 150.0 |
| Microcrystalline Cellulose NF | 41.50 | 124.5 |
| Hydroxypropyl Methylcellulose 2910, USP | 34.00 | 102.0 |
| Cysteine Hydrochloride, USP | 8.100 | 24.30 |
| Magnesium Stearate, NF | 1.400 | 4.200 |
| Purified Water. USP | q.s | q.s |
| | $\overline{135.0mg}$ | |
| Surface Area to Volume Ratio | $14.37cm^{-1}$ | |
| Volume | $0.1386cm^3$ | |
| Surface Area | $1.9914cm^2$ | |
| COATING INGREDIENTS | | |
| Opadry White YS-1-7059 | 5.000 | 20.00* |
| Purified Water, USP | q.s | 180.0 |
| Carnauba Wax, NF | 0.01333 | 0.040 |
| | $\overline{140.0mg}$ | |
| Coating thickness $\approx$ 0.03 - 0.07mm | | |

*includes 33.3*% overage

TYPICAL MANUFACTURING PROCEDURE AND ASSEMBLING PROCESS

Bupropion Hydrochloride Sustained-Release Tablets, 50mg Bupropion Hydrochloride Per Coated Tablet

1. Sift and blend the following:

    bupropion hydrochloride
    microcrystalline cellulose
    hydroxypropyl methylcellulose.

2. Dissolve the cysteine hydrochloride in the purified water.

3. Spray the solution from Step 2 onto the blended powders from Step 1 using a fluid bed granulator. Additional purified water may be used, if necessary, to obtain proper granule wetness.

8

4. Dry the granules.

5. Add magnesium stearate to the granules and mill.

6. Blend the milled granules.

7. Compress the granules at approximately 135.0mg or a weight equivalent to 50mg bupropion hydrochloride on a rotary press fitted with 7.4mm round punches and dies, the upper and lower punches plain.

8. Prepare a film-coating suspension of Opadry White, YS-1-7059 dispersed in purified water.

9. Spray the coating suspension onto the core tablets using suitable coating equipment until the desired weight gain is achieved.

10. Polish the coated tablets in the coating equipment using carnauba wax.

## Claims

**Claims for the following Contracting States : AT, BE, CH, DE, DK, FR, GB, IE, IT, LI, LU, MC, NL, PT, SE**

1. A sustained release tablet comprising bupropion hydrochloride and hydroxypropyl methylcellulose, the amount of hydroxypropyl methylcellulose to the amount of bupropion hydrochloride being from 0.19:1 to 1.1:1 respectively and the tablet being capable in water of releasing from 20 to 60 percent of the total amount of bupropion hydrochloride in 1 hour, from 50 to 95 percent of the total amount of bupropion hydrochloride in 4 hours and not less than 75 percent of the total amount of bupropion hydrochloride in 8 hours.

2. A sustained release tablet according to claim 1, wherein the amount of hydroxypropyl methylcellulose to the amount of bupropion hydrochloride is 0.26:1 to 0.68:1, the tablet being capable in water of releasing from 25 to 50 percent of the total amount of bupropion hydrochloride in 1 hour and from 60 to 95 percent of the total amount of bupropion hydrochloride in 4 hours.

3. A sustained release tablet according to either of claims 1 and 2, wherein bupropion hydrochloride is present in an amount of from 25 to 500 mg.

4. A sustained release tablet according to claim 3, containing from 50 to 150 mg of bupropion hydrochloride.

5. A sustained release tablet according to any of claims 1 to 4, wherein said tablet has a surface area to volume ratio of 3:1 to 25:1 $cm^{-1}$.

6. A sustained release tablet according to claim 5, wherein said surface area to volume ratio is 7:1 to 16:1 $cm^{-1}$.

7. A sustained release tablet according to any of the preceding claims, comprising glycine hydrochloride or cysteine hydrochloride.

8. A sustained release tablet according to any of the preceding claims, wherein said tablet has less than 10% degradation of bupropion hydrochloride when stored for one year at 15 to 25°C and 35% to 60% relative humidity.

9. A sustained release tablet according to any preceding claims, containing 50mg of bupropion hydrochloride and which releases between 30 to 50 percent of bupropion hydrochloride in 1 hour, 70 to 95 percent in 4 hours and not less than 80 percent in 8 hours, said tablet having a surface area to volume ratio of 13:1 to 16:1$cm^{-1}$.

10. A sustained release tablet according to any of claims 1 to 8, containing 100mg of bupropion hydrochloride and which releases between 25 to 45 percent of bupropion hydrochloride in 1 hour, 60 to 85 percent in 4 hours and not less than 80 percent in 8 hours, said tablet having a surface area to volume ratio of 9:1 to 12:1$cm^{-1}$.

11. A sustained release tablet according to any of claims 1 to 8, containing 150mg of bupropion hydrochloride and which releases 25 to 45 percent of bupropion hydrochloride in 1 hour, 60 to 75 percent in 4 hours and not less than 85 percent in 8 hours, said tablet having a surface area to volume ratio of 7:1 to 10:1$cm^{-1}$.

**12.** A sustained release tablet comprising 100mg of bupropion hydrochloride, 19 to 110mg of hydroxypropyl methylcellulose and 2.7 to 27mg of cysteine hydrochloride or glycine hydrochloride.

**13.** A sustained release tablet according to any of the preceding claims, for use in the treatment of depression in a human being.

**Claims for the following Contracting States : ES, GR**

**1.** A sustained release tablet comprising bupropion hydrochloride and hydroxypropyl methylcellulose, the amount of hydroxypropyl methylcellulose to the amount of bupropion hydrochloride being from 0.19:1 to 1.1:1 respectively and the tablet being capable in water of releasing from 20 to 60 percent of the total amount of bupropion hydrochloride in 1 hour, from 50 to 95 percent of the total amount of bupropion hydrochloride in 4 hours and not less than 75 percent of the total amount of bupropion hydrochloride in 8 hours.

**2.** A sustained release tablet according to claim 1, wherein the amount of hydroxypropyl methylcellulose to the amount of bupropion hydrochloride is 0.26:1 to 0.68:1, the tablet being capable in water of releasing from 25 to 50 percent of the total amount of bupropion hydrochloride in 1 hour and from 60 to 95 percent of the total amount of bupropion hydrochloride in 4 hours.

**3.** A sustained release tablet according to either of claims 1 and 2, wherein bupropion hydrochloride is present in an amount of from 25 to 500 mg.

**4.** A sustained release tablet according to claim 3, containing from 50 to 150 mg of bupropion hydrochloride.

**5.** A sustained release tablet according to any of claims 1 to 4, wherein said tablet has a surface area to volume ratio of 3:1 to 25:1 cm$^{-1}$.

**6.** A sustained release tablet according to claim 5, wherein said surface area to volume ratio is 7:1 to 16:1 cm$^{-1}$.

**7.** A sustained release tablet according to any of the preceding claims, comprising glycine hydrochloride or cysteine hydrochloride.

**8.** A sustained release tablet according to any of the preceding claims, wherein said tablet has less than 10% degradation of bupropion hydrochloride when stored for one year at 15 to 25°C and 35% to 60% relative humidity.

**9.** A sustained release tablet according to any preceding claim, containing 50mg of bupropion hydrochloride and which releases between 30 to 50 percent of bupropion hydrochloride in 1 hour, 70 to 95 percent in 4 hours and not less than 80 percent in 8 hours, said tablet having a surface area to volume ratio of 13:1 to 16:1cm$^{-1}$.

**10.** A sustained release tablet according to any of claims 1 to 8, containing 100mg of bupropion hydrochloride and which releases between 25 to 45 percent of bupropion hydrochloride in 1 hour, 60 to 85 percent in 4 hours and not less than 80 percent in 8 hours, said tablet having a surface area to volume ratio of 9:1 to 12:1cm$^{-1}$.

**11.** A sustained release tablet according to any of claims 1 to 8, containing 150mg of bupropion hydrochloride and which releases 25 to 45 percent of bupropion hydrochloride in 1 hour, 60 to 75 percent in 4 hours and not less than 85 percent in 8 hours, said tablet having a surface area to volume ratio of 7:1 to 10:1cm$^{-1}$.

**12.** A sustained release tablet comprising 100mg of bupropion hydrochloride, 19 to 110mg of hydroxypropyl methylcellulose and 2.7 to 27mg of cysteine hydrochloride or glycine hydrochloride.

**13.** A sustained release tablet according to any of the preceding claims, for use in the treatment of depression in a human being.

**14.** A process of preparing a sustained release tablet comprising bupropion hydrochloride and hydroxypropyl methylcellulose, the amount of hydroxypropyl methylcellulose to the amount of bupropion hydrochloride being from 0.19:1 to 1.1:1 respectively and the tablet being capable in water of releasing from 20 to 60 percent of the total amount of bupropion hydrochloride in 1 hour, from 50 to 95 percent of the total amount of bupropion hydrochloride in 4 hours and not less than 75 percent of the total amount of bupropion hydrochloride in 8 hours.

**15.** A process according to claim 14, wherein the amount of hydroxypropyl methylcellulose to the amount of bupropion hydrochloride is 0.26:1 to 0.68:1, the tablet being capable in water of releasing from 25 to 50 percent of the total amount of bupropion hydrochloride in 1 hour and from 60 to 95 percent of the total amount of bupropion in 4 hours.

**16.** A process according to claim 14 or 15, wherein bupropion hydrochloride is present in an amount of from 25 to 500 mg.

**17.** A process according to claim 16, containing from 50 to 150 mg of bupropion hydrochloride.

**18.** A process according to any of claims 14 to 17, wherein said tablet has a surface area to volume ratio of 3:1 to 25:1 $cm^{-1}$.

**19.** A process according to claim 18, wherein said surface area to volume ratio is 7:1 to 16:1 $cm^{-1}$.

**20.** A process according to any of claims 14 to 19, which further comprises glycine hydrochloride or cysteine hydrochloride.

**21.** A process according to any of claims 14 to 20, wherein said tablet has less than 10% degradation of bupropion hydrochloride when stored for one year at 15 to 25°C and 35% to 60% relative humidity.

**22.** A process according to any of claims 14 to 21, wherein said tablet contains 50mg of bupropion hydrochloride and releases between 30 to 50 percent of bupropion hydrochloride in 1 hour, 70 to 95 percent in 4 hours and not less than 80 percent in 8 hours, said tablet having a surface area to volume ratio of 13:1 to 16:1$cm^{-1}$.

**23.** A process according to any of claims 14 to 21, wherein said tablet contains 100mg of bupropion hydrochloride and releases between 25 to 45 percent of bupropion hydrochloride in 1 hour, 60 to 85 percent in 4 hours and not less than 80 percent in 8 hours, said tablet having a surface area to volume ratio of 9:1 to 12:1$cm^{-1}$.

**24.** A process according to any of claims 14 to 21, wherein said tablet contains 150mg of bupropion hydrochloride and releases 25 to 45 percent of bupropion hydrochloride in 1 hour, 60 to 75 percent in 4 hours and not less than 85 percent in 8 hours, said tablet having a surface area to volume ratio of 7:1 to 10:1$cm^{-1}$.

**25.** A process of preparing a sustained release tablet comprising 100mg of bupropion hydrochloride, 19 to 110mg of hydroxypropyl methylcellulose and 2.7 to 27mg of cysteine hydrochloride or glycine hydrochloride.

**Patentansprüche**

**Patentansprüche für folgende Vertragsstaaten : AT, BE, CH, DE, DK, FR, GB, IE, IT, LI, LU, MC, NL, PT, SE**

**1.** Tablette mit verzögerter Freisetzung, enthaltend Bupropionhydrochlorid und Hydroxypropylmethylcellulose, wobei die Menge an Hydroxypropylmethylcellulose zu der Menge an Bupropionhydrochlorid 0,19:1 bis 1,1:1 beträgt und die Tablette in Wasser 20 bis 60% der Gesamtmenge an Bupropionhydrochlorid in 1 Stunde, 50 bis 95% der Gesamtmenge an Bupropionhydrochlorid in 4 Stunden und nicht weniger als 75% der Gesamtmenge an Bupropionhydrochlorid in 8 Stunden freisetzt.

**2.** Tablette mit verzögerter Freisetzung nach Anspruch 1, dadurch **gekennzeichnet,** daß die Menge an Hydroxypropylmethylcellulose zu der Menge an Bupropionhydrochlorid 0,26:1 bis 0,68:1 beträgt und die Tablette in Wasser 25 bis 50% der Gesamtmenge an Bupropionhydrochlorid in 1 Stunde und 60 bis 95% der Gesamtmenge an Bupropionhydrochlorid in 4 Stunden freisetzt.

**3.** Tablette mit verzögerter Freisetzung nach den Ansprüchen 1 und 2, dadurch **gekennzeichnet,** daß das Bupropionhydrochlorid in einer Menge von 25 bis 500 mg vorhanden ist.

**4.** Tablette mit verzögerter Freisetzung nach Anspruch 3, dadurch **gekennzeichnet,** daß sie 50 bis 150 mg Bupropionhydrochlorid enthält.

**5.** Tablette mit verzögerter Freisetzung nach einem der Ansprüche 1 bis 4, dadurch **gekennzeichnet,** daß die Tablette ein Oberflächen-zu-Volumen-Verhältnis von 3:1 bis 25:1 $cm^{-1}$ besitzt.

6. Tablette mit verzögerter Freisetzung nach Anspruch 5, dadurch **gekennzeichnet,** daß das Oberflächenzu-Volumen-Verhältnis 7:1 bis 16:1 $cm^{-1}$ beträgt.

7. Tablette mit verzögerter Freisetzung nach einem der vorhergehenden Ansprüche, dadurch **gekennzeichnet,** daß sie Glycinhydrochlorid oder Cysteinhydrochlorid enthält.

8. Tablette mit verzögerter Freisetzung nach einem der vorhergehenden Ansprüche, dadurch **gekennzeichnet,** daß die Tablette weniger als 10% Abbau von Bupropionhydrochlorid zeigt, wenn sie während eines Jahres bei 15 bis 25°C und 35 bis 60% relativer Feuchtigkeit gelagert wird.

9. Tablette mit verzögerter Freisetzung nach einem der vorhergehenden Ansprüche, dadurch **gekennzeichnet,** daß sie 50 mg Bupropionhydrochlorid enthält und zwischen 30 bis 50% Bupropionhydrochlorid in 1 Stunde, 70 bis 95% in 4 Stunden und nicht weniger als 80% in 8 Stunden freisetzt, wobei die Tablette ein Oberflächen-zu-Volumen-Verhältnis von 13:1 bis 16:1 $cm^{-1}$ besitzt.

10. Tablette mit verzögerter Freisetzung nach einem der Ansprüche 1 bis 8, dadurch **gekennzeichnet,** daß sie 100 mg Bupropionhydrochlorid enthält und zwischen 25 bis 45% Bupropionhydrochlorid in 1 Stunde, 60 bis 85% in 4 Stunden und nicht weniger als 80% in 8 Stunden freisetzt, wobei die Tablette ein Oberflächen-zu-Volumen-Verhältnis von 9:1 bis 12:1 $cm^{-1}$ besitzt.

11. Tablette mit verzögerter Freisetzung nach einem der Ansprüche 1 bis 8, dadurch **gekennzeichnet,** daß sie 150 mg Bupropionhydrochlorid enthält und 25 bis 45% Bupropionhydrochlorid in 1 Stunde, 60 bis 75% in 4 Stunden und nicht weniger als 85% in 8 Stunden freisetzt, wobei die Tablette ein Oberflächen-zu-Volumen-Verhältnis von 7:1 bis 10:1 $cm^{-1}$ besitzt.

12. Tablette mit verzögerter Freisetzung, enthaltend 100 mg Bupropionhydrochlorid, 19 bis 110 mg Hydroxypropylmethylcellulose und 2,7 bis 27 mg Cysteinhydrochlorid oder Glycinhydrochlorid.

13. Tablette mit verzögerter Freisetzung nach irgendeinem der vorhergehenden Ansprüche für die Verwendung bei der Behandlung von Depressionen bei Menschen.

**Patentansprüche für folgende Vertragsstaaten : ES, GR**

1. Tablette mit verzögerter Freisetzung, enthaltend Bupropionhydrochlorid und Hydroxypropylmethylcellulose, wobei die Menge an Hydroxypropylmethylcellulose zu der Menge an Bupropionhydrochlorid 0,19:1 bis 1,1:1 beträgt und die Tablette in Wasser 20 bis 60% der Gesamtmenge an Bupropionhydrochlorid in 1 Stunde, 50 bis 95% der Gesamtmenge an Bupropionhydrochlorid in 4 Stunden und nicht weniger als 75% der Gesamtmenge an Bupropionhydrochlorid in 8 Stunden freisetzt.

2. Tablette mit verzögerter Freisetzung nach Anspruch 1, dadurch **gekennzeichnet,** daß die Menge an Hydroxypropylmethylcellulose zu der Menge an Bupropionhydrochlorid 0,26:1 bis 0,68:1 beträgt und die Tablette in Wasser 25 bis 50% der Gesamtmenge an Bupropionhydrochlorid in 1 Stunde und 60 bis 95% der Gesamtmenge an Bupropionhydrochlorid in 4 Stunden freisetzt.

3. Tablette mit verzögerter Freisetzung nach den Ansprüchen 1 und 2, dadurch **gekennzeichnet,** daß das Bupropionhydrochlorid in einer Menge von 25 bis 500 mg vorhanden ist.

4. Tablette mit verzögerter Freisetzung nach Anspruch 3, dadurch **gekennzeichnet,** daß sie 50 bis 150 mg Bupropionhydrochlorid enthält.

5. Tablette mit verzögerter Freisetzung nach einem der Ansprüche 1 bis 4, dadurch **gekennzeichnet,** daß die Tablette ein Oberflächen-zu-Volumen-Verhältnis von 3:1 bis 25:1 $cm^{-1}$ besitzt.

6. Tablette mit verzögerter Freisetzung nach Anspruch 5, dadurch **gekennzeichnet,** daß das Oberflächenzu-Volumen-Verhältnis 7:1 bis 16:1 $cm^{-1}$ beträgt.

7. Tablette mit verzögerter Freisetzung nach einem der vorhergehenden Ansprüche, dadurch **gekennzeichnet,** daß sie Glycinhydrochlorid oder Cysteinhydrochlorid enthält.

**8.** Tablette mit verzögerter Freisetzung nach einem der vorhergehenden Ansprüche, dadurch **gekennzeichnet,** daß die Tablette weniger als 10% Abbau von Bupropionhydrochlorid zeigt, wenn sie während eines Jahres bei 15 bis 25°C und 35 bis 60% relativer Feuchtigkeit gelagert wird.

**9.** Tablette mit verzögerter Freisetzung nach einem der vorhergehenden Ansprüche, dadurch **gekennzeichnet,** daß sie 50 mg Bupropionhydrochlorid enthält und zwischen 30 bis 50% Bupropionhydrochlorid in 1 Stunde, 70 bis 95% in 4 Stunden und nicht weniger als 80% in 8 Stunden freisetzt, wobei die Tablette ein Oberflächen-zu-Volumen-Verhältnis von 13:1 bis 16:1 cm$^{-1}$ besitzt.

**10.** Tablette mit verzögerter Freisetzung nach einem der Ansprüche 1 bis 8, dadurch **gekennzeichnet,** daß sie 100 mg Bupropionhydrochlorid enthält und zwischen 25 bis 45% Bupropionhydrochlorid in 1 Stunde, 60 bis 85% in 4 Stunden und nicht weniger als 80% in 8 Stunden freisetzt, wobei die Tablette ein Oberflächen-zu-Volumen-Verhältnis von 9:1 bis 12:1 cm$^{-1}$ besitzt.

**11.** Tablette mit verzögerter Freisetzung nach einem der Ansprüche 1 bis 8, dadurch **gekennzeichnet,** daß sie 150 mg Bupropionhydrochlorid enthält und 25 bis 45% Bupropionhydrochlorid in 1 Stunde, 60 bis 75% in 4 Stunden und nicht weniger als 85% in 8 Stunden freisetzt, wobei die Tablette ein Oberflächen-zu-Volumen-Verhältnis von 7:1 bis 10:1 cm$^{-1}$ besitzt.

**12.** Tablette mit verzögerter Freisetzung, enthaltend 100 mg Bupropionhydrochlorid, 19 bis 110 mg Hydroxypropylmethylcellulose und 2,7 bis 27 mg Cysteinhydrochlorid oder Glycinhydrochlorid.

**13.** Tablette mit verzögerter Freisetzung nach irgendeinem der vorhergehenden Ansprüche für die Verwendung bei der Behandlung von Depressionen bei Menschen.

**14.** Verfahren zur Herstellung einer Tablette mit verzögerter Freisetzung, enthaltend Bupropionhydrochlorid und Hydroxypropylmethylcellulose, wobei die Menge an Hydroxypropylmethylcellulose zu der Menge an Bupropionhydrochlorid 0,19:1 bis 1,1:1 beträgt und die Tablette in Wasser 20 bis 60% der Gesamtmenge an Bupropionhydrochlorid in 1 Stunde, 50 bis 95% der Gesamtmenge an Bupropionhydrochlorid in 4 Stunden und nicht weniger als 75% der Gesamtmenge an Bupropionhydrochlorid in 8 Stunden freisetzt.

**15.** Verfahren nach Anspruch 14, dadurch **gekennzeichnet,** daß die Menge an Hydroxypropylmethylcellulose zu der Menge an Bupropionhydrochlorid 0,26:1 bis 0,68:1 beträgt und die Tablette in Wasser 25 bis 50% der Gesamtmenge an Bupropionhydrochlorid in 1 Stunde und 60 bis 95% der Gesamtmenge an Bupropionhydrochlorid in 4 Stunden freisetzt.

**16.** Verfahren nach Anspruch 14 oder 15, dadurch **gekennzeichnet,** daß das Bupropionhydrochlorid in einer Menge von 25 bis 500 mg vorhanden ist.

**17.** Verfahren nach Anspruch 16, dadurch **gekennzeichnet,** daß die Tablette 50 bis 150 mg Bupropionhydrochlorid enthält.

**18.** Verfahren nach einem der Ansprüche 14 bis 17, dadurch **gekennzeichnet,** daß die Tablette ein Oberflächen-zu-Volumen-Verhältnis von 3:1 bis 25:1 cm$^{-1}$ besitzt.

**19.** Verfahren nach Anspruch 18, dadurch **gekennzeichnet,** daß das Oberflächen-zu-Volumen-Verhältnis 7:1 bis 16:1 cm$^{-1}$ beträgt.

**20.** Verfahren nach einem der Ansprüche 14 bis 19, dadurch **gekennzeichnet,** daß die Tablette Glycinhydrochlorid oder Cysteinhydrochlorid enthält.

**21.** Verfahren nach einem der Ansprüche 14 bis 20, dadurch **gekennzeichnet,** daß die Tablette weniger als 10% Abbau von Bupropionhydrochlorid zeigt, wenn sie während eines Jahres bei 15 bis 25°C und 35 bis 60% relativer Feuchtigkeit gelagert wird.

**22.** Verfahren nach einem der Ansprüche 14 bis 21, dadurch **gekennzeichnet,** daß die Tablette 50 mg Bupropionhydrochlorid enthält und zwischen 30 bis 50% Bupropionhydrochlorid in 1 Stunde, 70 bis 95% in 4 Stunden und nicht weniger als 80% in 8 Stunden freisetzt, wobei die Tablette ein Oberflächen-zu-Volumen-Verhältnis von 13:1 bis

16:1 cm$^{-1}$ besitzt.

23. Verfahren nach einem der Ansprüche 14 bis 21, dadurch **gekennzeichnet,** daß die Tablette 100 mg Bupropionhydrochlorid enthält und zwischen 25 bis 45% Bupropionhydrochlorid in 1 Stunde, 60 bis 85% in 4 Stunden und nicht weniger als 80% in 8 Stunden freisetzt, wobei die Tablette ein Oberflächen-zu-Volumen-Verhältnis von 9:1 bis 12:1 cm$^{-1}$ besitzt.

24. Verfahren nach einem der Ansprüche 14 bis 21, dadurch **gekennzeichnet,** daß die Tablette 150 mg Bupropionhydrochlorid enthält und 25 bis 45% Bupropionhydrochlorid in 1 Stunde, 60 bis 75% in 4 Stunden und nicht weniger als 85% in 8 Stunden freisetzt, wobei die Tablette ein Oberflächen-zu-Volumen-Verhältnis von 7:1 bis 10:1 cm$^{-1}$ besitzt.

25. Verfahren zur Herstellung einer Tablette mit verzögerter Freisetzung, enthaltend 100 mg Bupropionhydrochlorid, 19 bis 110 mg Hydroxypropylmethylcellulose und 2,7 bis 27 mg Cysteinhydrochlorid oder Glycinhydrochlorid.

**Revendications**

**Revendications pour les Etats contractants suivants : AT, BE, CH, DE, DK, FR, GB, IE, IT, LI, LU, MC, NL, PT, SE**

1. Comprimé à libération soutenue comprenant du chlorhydrate de bupropion et de l'hydroxypropylméthylcellulose, la proportion d'hydroxypropylméthylcellulose, par rapport à celle du chlorhydrate de bupropion variant de 0,19:1 à 1,1:1, respectivement et le comprimé est capable de libérer dans l'eau de 20 à 60% de la proportion totale du chlorhydrate de bupropion, en l'espace d'une heure, de 50 à 95% de la proportion totale du chlorhydrate de bupropion, en l'espace de 4 heures et pas moins de 75% de la proportion totale du chlorhydrate de bupropion, en l'espace de 8 heures.

2. Comprimé à libération soutenue suivant la revendication 1, caractérisé en ce que la proportion d'hydroxypropylméthylcellulose, par rapport à la proportion de chlorhydrate de bupropion varie de 0,26:1 à 0,68:1, le comprimé étant capable de libérer, dans l'eau, de 25 à 50% de la proportion totale de chlorhydrate de bupropion en l'espace d'une heure et de 60 à 95% de la proportion totale de chlorhydrate de bupropion en l'espace de 4 heures.

3. Comprimé à libération soutenue suivant l'une quelconque des revendications 1 et 2, caractérisé en ce que le chlorhydrate de bupropion y est présent en une proportion de 25 à 500 mg.

4. Comprimé à libération soutenue suivant la revendication 3, caractérisé en ce qu'il contient de 50 à 150 mg de chlorhydrate de bupropion.

5. Comprimé à libération soutenue suivant l'une quelconque des revendications 1 à 4, caractérisé en ce qu'il possède un rapport surface à volume de 3:1 à 25:1 cm$^{-1}$.

6. Comprimé à libération soutenue suivant la revendication 5, caractérisé en ce que le rapport surface à volume varie de 7:1 à 16:1 cm$^{-1}$.

7. Comprimé à libération soutenue suivant l'une quelconque des revendications précédentes, caractérisé en ce qu'il comprend du chlorhydrate de glycine ou du chlorhydrate de cystéine.

8. Comprimé à libération soutenue suivant l'une quelconque des revendications précédentes, caractérisé en ce qu'il présente moins de 10% de dégradation de chlorhydrate de bupropion lorsqu'il est conservé pendant une année à 15-25°C, sous une humidité relative de 35% à 60%.

9. Comprimé à libération soutenue suivant l'une quelconque des revendications précédentes, caractérisé en ce qu'il contient 50 mg de chlorhydrate de bupropion et qu'il libère de 30 à 50% de chlorhydrate de bupropion, en l'espace d'une heure, de 70 à 95% de chlorhydrate de bupropion, en l'espace de 4 heures et pas moins de 80% de chlorhydrate de bupropion, en l'espace de 8 heures, ce comprimé possédant un rapport surface à volume de 13:1 à 16:1 cm$^{-1}$.

10. Comprimé à libération soutenue suivant l'une quelconque des revendications 1 à 8, caractérisé en ce qu'il contient 100 mg de chlorhydrate de bupropion et qu'il libère de 25 à 45% de chlorhydrate de bupropion, en l'espace d'une heure, de 60 à 85% de chlorhydrate de bupropion, en l'espace de 4 heures et pas moins de 80% de chlorhydrate de bupropion, en l'espace de 8 heures, ce comprimé possédant un rapport surface à volume de 9:1 à 12:1 cm$^{-1}$.

11. Comprimé à libération soutenue suivant l'une quelconque des revendications 1 à 8, caractérisé en ce qu'il contient 150 mg de chlorhydrate de bupropion et qu'il libère de 25 à 45% de chlorhydrate de bupropion, en l'espace d'une heure, de 60 à 75% de chlorhydrate de bupropion, en l'espace de 4 heures et pas moins de 85% de chlorhydrate de bupropion, en l'espace de 8 heures, ce comprimé possédant un rapport surface à volume de 7:1 à 10:1 cm$^{-1}$.

12. Comprimé à libération soutenue, caractérisé en ce qu'il comprend 100 mg de chlorhydrate de bupropion, 19 à 110 mg d'hydroxypropylméthylcellulose et 2,7 à 27 mg de chlorhydrate de cystéine ou de chlorhydrate de glycine.

13. Comprimé à libération soutenue suivant l'une quelconque des revendications précédentes, à utiliser pour le traitement de la dépression chez un être humain.

**Revendications pour les Etats contractants suivants : ES, GR**

1. Comprimé à libération soutenue comprenant du chlorhydrate de bupropion et de l'hydroxypropylméthylcellulose, la proportion d'hydroxypropylméthylcellulose, par rapport à celle du chlorhydrate de bupropion variant de 0,19:1 à 1,1:1, respectivement et le comprimé est capable de libérer dans l'eau de 20 à 60% de la proportion totale du chlorhydrate de bupropion, en l'espace d'une heure, de 50 à 95% de la proportion totale du chlorhydrate de bupropion, en l'espace de 4 heures et pas moins de 75% de la proportion totale du chlorhydrate de bupropion, en l'espace de 8 heures.

2. Comprimé à libération soutenue suivant la revendication 1, caractérisé en ce que la proportion d'hydroxypropylméthylcellulose, par rapport à la proportion de chlorhydrate de bupropion varie de 0,26:1 à 0,68:1, le comprimé étant capable de libérer, dans l'eau, de 25 à 50% de la proportion totale de chlorhydrate de bupropion en l'espace d'une heure et de 60 à 95% de la proportion totale de chlorhydrate de bupropion en l'espace de 4 heures.

3. Comprimé à libération soutenue suivant l'une quelconque des revendications 1 et 2, caractérisé en ce que le chlorhydrate de bupropion y est présent en une proportion de 25 à 500 mg.

4. Comprimé à libération soutenue suivant la revendication 3, caractérisé en ce qu'il contient de 50 à 150 mg de chlorhydrate de bupropion.

5. Comprimé à libération soutenue suivant l'une quelconque des revendications 1 à 4, caractérisé en ce qu'il possède un rapport surface à volume de 3:1 à 25:1 cm$^{-1}$.

6. Comprimé à libération soutenue suivant la revendication 5, caractérisé en ce que le rapport surface à volume varie de 7:1 à 16:1 cm$^{-1}$.

7. Comprimé à libération soutenue suivant l'une quelconque des revendications précédentes, caractérisé en ce qu'il comprend du chlorhydrate de glycine ou du chlorhydrate de cystéine.

8. Comprimé à libération soutenue suivant l'une quelconque des revendications précédentes, caractérisé en ce qu'il présente moins de 10% de dégradation de chlorhydrate de bupropion lorsqu'il est conservé pendant une année à 15-25°C, sous une humidité relative de 35% à 60%.

9. Comprimé à libération soutenue suivant l'une quelconque des revendications précédentes, caractérisé en ce qu'il contient 50 mg de chlorhydrate de bupropion et qu'il libère de 30 à 50% de chlorhydrate de bupropion, en l'espace d'une heure, de 70 à 95% de chlorhydrate de bupropion, en l'espace de 4 heures et pas moins de 80% de chlorhydrate de bupropion, en l'espace de 8 heures, ce comprimé possédant un rapport surface à volume de 13:1 à 16:1 cm$^{-1}$.

10. Comprimé à libération soutenue suivant l'une quelconque des revendications 1 à 8, caractérisé en ce qu'il contient 100 mg de chlorhydrate de bupropion et qu'il libère de 25 à 45% de chlorhydrate de bupropion, en l'espace d'une heure, de 60 à 85% de chlorhydrate de bupropion, en l'espace de 4 heures et pas moins de 80% de chlorhydrate de bupropion, en l'espace de 8 heures, ce comprimé possédant un rapport surface à volume de 9:1 à 12:1 cm$^{-1}$.

**11.** Comprimé à libération soutenue suivant l'une quelconque des revendications 1 à 8, caractérisé en ce qu'il contient 150 mg de chlorhydrate de bupropion et qu'il libère de 25 à 45% de chlorhydrate de bupropion, en l'espace d'une heure, de 60 à 75% de chlorhydrate de bupropion, en l'espace de 4 heures et pas moins de 85% de chlorhydrate de bupropion, en l'espace de 8 heures, ce comprimé possédant un rapport surface à volume de 7:1 à 10:1 cm$^{-1}$.

**12.** Comprimé à libération soutenue, caractérisé en ce qu'il comprend 100 mg de chlorhydrate de bupropion, 19 à 110 mg d'hydroxypropylméthylcellulose et 2,7 à 27 mg de chlorhydrate de cystéine ou de chlorhydrate de glycine.

**13.** Comprimé à libération soutenue suivant l'une quelconque des revendications précédentes, à utiliser pour le traitement de la dépression chez un être humain.

**14.** Procédé de préparation d'un comprimé à libération soutenue, comprenant du chlorhydrate de bupropion et de l'hydroxypropylméthylcellulose, le rapport de la proportion de l'hydroxypropylméthylcellulose à la proportion du chlorhydrate de bupropion variant de 0,19:1 à 1,1:1, respectivement et le comprimé étant capable, dans l'eau, de libérer de 20 à 60% de la proportion totale de chlorhydrate de bupropion en l'espace d'une heure, de 50 à 95% de la proportion totale de chlorhydrate de bupropion, en l'espace de 4 heures et pas moins de 75% de la proportion totale de chlorhydrate de bupropion, en l'espace de 8 heures.

**15.** Procédé suivant la revendication 14, caractérisé en ce que la proportion d'hydroxypropylméthylcellulose, par rapport à la proportion de chlorhydrate de bupropion varie de 0,26:1 à 0,68:1, le comprimé étant capable de libérer, dans l'eau, de 25 à 50% de la proportion totale de chlorhydrate de bupropion en l'espace d'une heure et de 60 à 95% de la proportion totale de chlorhydrate de bupropion en l'espace de 4 heures.

**16.** Procédé suivant la revendication 14 ou 15, caractérisé en ce que le chlorhydrate bupropion y est présent en une proportion de 25 à 500 mg.

**17.** Procédé suivant la revendication 16, caractérisé en ce qu'il contient de 50 à 150 mg de chlorhydrate de bupropion.

**18.** Procédé suivant l'une quelconque des revendications 14 à 17, caractérisé en ce qu'il possède un rapport surface à volume de 3:1 à 25:1 cm$^{-1}$.

**19.** Procédé suivant la revendication 18, caractérisé en ce que le rapport surface à volume varie de 7:1 à 16:1 cm$^{-1}$.

**20.** Procédé suivant l'une quelconque des revendications 14 à 19, caractérisé en ce qu'il comprend du chlorhydrate de glycine ou du chlorhydrate de cystéine.

**21.** Procédé suivant l'une quelconque des revendications 14 à 20, caractérisé en ce qu'il présente moins de 10% de dégradation de chlorhydrate de bupropion lorsqu'il est conservé pendant une année à 15-25°C, sous une humidité relative de 35% à 60%.

**22.** Procédé suivant l'une quelconque des revendications 14 à 21, caractérisé en ce qu'il contient 50 mg de chlorhydrate de bupropion et qu'il libère de 30 à 50% de chlorhydrate de bupropion, en l'espace d'une heure, de 70 à 95% de chlorhydrate de bupropion, en l'espace de 4 heures et pas moins de 80% de chlorhydrate de bupropion, en l'espace de 8 heures, ce comprimé possédant un rapport surface à volume de 13:1 à 16:1 cm$^{-1}$.

**23.** Procédé suivant l'une quelconque des revendications 14 à 21, caractérisé en ce qu'il contient 100 mg de chlorhydrate de bupropion et qu'il libère de 25 à 45% de chlorhydrate de bupropion, en l'espace d'une heure, de 60 à 85% de chlorhydrate de bupropion, en l'espace de 4 heures et pas moins de 80% de chlorhydrate de bupropion, en l'espace de 8 heures, ce comprimé possédant un rapport surface à volume de 9:1 à 12:1 cm$^{-1}$.

**24.** Procédé suivant l'une quelconque des revendications 14 à 21, caractérisé en ce qu'il contient 150 mg de chlorhydrate de bupropion et qu'il libère de 25 à 45% de chlorhydrate de bupropion, en l'espace d'une heure, de 60 à 75% de chlorhydrate de bupropion, en l'espace de 4 heures et pas moins de 85% de chlorhydrate de bupropion, en l'espace de 8 heures, ce comprimé possédant un rapport surface à volume de 7:1 à 10:1 cm$^{-1}$.

**25.** Procédé de préparation d'un comprimé à libération soutenue, caractérisé en ce qu'il comprend 100 mg de chlorhydrate de bupropion, 19 à 110 mg d'hydroxypropylméthylcellulose et 2,7 à 27 mg de chlorhydrate de cystéine ou de chlorhydrate de glycine.

FIG. 1

FIG. 2

FIG. 3

FIG. 4

EP 0 656 775 B1

FIG. 5

FIG. 6

EP 0 656 775 B1